(19) Europäisches Patentamt
European Patent Office
Office européen des brevets

(11) EP 4 285 903 A1

(12) EUROPEAN PATENT APPLICATION
published in accordance with Art. 153(4) EPC

(43) Date of publication:
06.12.2023 Bulletin 2023/49

(21) Application number: 22745250.5

(22) Date of filing: 26.01.2022

(51) International Patent Classification (IPC):
*A61K 31/4709* (2006.01) *A61K 31/4152* (2006.01)
*A61P 9/10* (2006.01)

(52) Cooperative Patent Classification (CPC):
A61K 31/4152; A61K 31/4709; A61P 9/10;
A61P 25/00

(86) International application number:
PCT/CN2022/073910

(87) International publication number:
WO 2022/161376 (04.08.2022 Gazette 2022/31)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 29.01.2021 CN 202110122555

(71) Applicant: Neurodawn Pharmaceutical Co., Ltd.
Jiangsu 211199 (CN)

(72) Inventors:
- HUA, Yao
Nanjing, Jiangsu 210046 (CN)
- WANG, Lei
Nanjing, Jiangsu 210046 (CN)
- ZHANG, Zhengping
Nanjing, Jiangsu 210046 (CN)
- CHEN, Rong
Nanjing, Jiangsu 210046 (CN)
- YANG, Shibao
Nanjing, Jiangsu 210046 (CN)

(74) Representative: Lavoix
Bayerstraße 83
80335 München (DE)

# (54) USE OF COMPOSITION CONTAINING VINPOCETINE IN CEREBROVASCULAR DISEASES

(57) Disclosed is the use of a composition containing cilostazol or a pharmaceutically acceptable salt thereof and edaravone in the preparation of a drug for treating cerebrovascular diseases, especially ischemic cerebrovascular diseases. The experiment result shows that the tail vein administration of 1-15 mg/kg of cilostazol or 1.67-8.33 mg/kg of edaravone on rats with a focal cerebral ischemia-reperfusion injury can significantly ameliorate neurological defects in rats with an MCAO and reduce a cerebral infarction area; and performing compound combination within the dosage range (the mass ratio of cilostazol: edaravone is 1:5 to 5:1) can be synergistic. The tail vein administration of 3.33-16.67 mg/kg of cilostazol or 3.33-16.67 mg/kg of edaravone on mice with a focal cerebral ischemia-reperfusion injury can significantly ameliorate neurological defects in mice with an MCAO and reduce a cerebral infarction area.

EP 4 285 903 A1

## Description

## CROSS REFERENCE TO RELATED APPLICATIONS

This application claims the priority of Chinese Patent Application No. 202110122555.3, filed with the China National Intellectual Property Administration on January 29, 2021, and titled with "USE OF COMPOSITION CONTAINING CILOSTAZOL IN CEREBROVASCULAR DISEASES", which is hereby incorporated by reference in its entirety.

## FIELD

The present invention belongs to the field of pharmacy, and relates to use of a composition of cilostazol and edaravone in the manufacture of a medicament for treating cerebrovascular diseases, in particular ischemic cerebrovascular diseases.

## BACKGROUND

Cerebrovascular disease (CVD) refers to brain lesions caused by various cerebrovascular diseases, and can be divided into acute cerebrovascular disease (stroke) and chronic cerebrovascular disease according to its pathogenesis process. Acute cerebrovascular diseases include transient ischemic attack, cerebral thrombosis, cerebral embolism, hypertensive encephalopathy, cerebral hemorrhage, subarachnoid hemorrhage, etc.; chronic cerebrovascular diseases include cerebral arteriosclerosis, cerebrovascular dementia, cerebral arterial steal syndrome, Parkinson’s disease, etc. Ischemic stroke is a general term for brain tissue necrosis caused by insufficient cerebral blood supply due to stenosis or occlusion of cerebral blood supply arteries (carotid artery and vertebral artery). Cerebral ischemia includes four types, transient ischemic attack (TIA), reversible neurological deficit (RIND), stroke in progressive (SIE) and complete stroke (CS). There is no cerebral infarction present in TIA, but there are cerebral infarction of different degrees present in RIND, SIE and CS.

Cilostazol is an anti-platelet aggregation drug, which was first developed and synthesized by Otsuka Pharmaceutical Co., Ltd., Japan, launched in Japan in 1988, approved by FDA in the United States in May 1999 and entered China in 1996. Cilostazol is a selective inhibitor of phosphodiesterase 3 (PDE3). The binding rate of cilostazol to plasma protein is about 95%, and most of cilostazol exist in a relatively stable prototype. Cilostazol has broad-spectrum pharmacological activity and has clinical value for many diseases, such as peripheral thrombotic disease and intermittent claudication. Moreover, cilostazol has the functions of anti-platelet and vasodilation, so that it can prevent the recurrence of circulatory shock and coronary artery stenosis. Studies have shown that PDE3 can inhibit the degradation of cAMP in the circulatory system, increase cAMP in platelets and vascular smooth muscle, inhibit the formation of platelets and promote the proliferation of vascular smooth muscle cells. Cilostazol inhibits the degradation of platelets mainly by affecting the following factors: arachidonic acid, adenosine diphosphate, epinephrine, collagen and fibrinase. At present, some experts believe that cilostazol treatment can be recommended for patients with carotid artery thrombosis, which can treat or prevent cerebral ischemia. Meanwhile, PDE3 can inhibit the production of nitric oxide synthase (NOS), thereby reducing the production of nitric oxide (NO).

Cilostazol has a structure represented by the formula as follows:

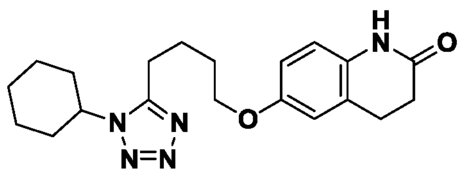

(molecular formula: $C_{20}H_{27}N_5O_2$; molecular weight: 369.47).

Edaravone (chemical name: 3-methyl-1-phenyl-2-pyrazolin-5-one) is a cerebral neuroprotective agent on the market (*Yakugaku Zasshi. 2004, 124(3): 99 -111*). Studies have shown that edaravone has antioxidant activity, and can significantly ameliorate the neurological deficit symptoms in animals with cerebral ischemia-reperfusion, reduce the area of cerebral infarction, reduce the degree of brain injury, alleviate cerebral edema, and inhibit lipid peroxidation in damaged brain tissue.

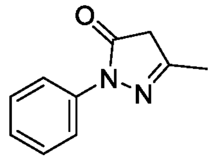

Edaravone

(molecular formula: $C_{10}H_{10}N_2O$; molecular weight: 174.20)

In summary, it is of great practical significance to provide a composition of cilostazol and edaravone for treating cerebrovascular diseases, especially ischemic cerebrovascular diseases.

## SUMMARY

An object of the present invention is to provide use of a pharmaceutical composition in the manufacture of a medicament for treating cerebrovascular diseases, wherein the pharmaceutical composition comprises cilostazol or a pharmaceutically acceptable salt thereof and edaravone. Further, the combined use of the pharmaceutical composition can synergistically increase the efficacy of treating cerebrovascular diseases.
In order to achieve the above object of the present invention, the present invention provides the following technical solutions:
In a first aspect, the present invention provides a composition comprising the following components:

component (I), cilostazol, a derivative, a pharmaceutically acceptable salt, or a prodrug molecule thereof; and

component (II), edaravone, or a drug with an active ingredient of edaravone.

In some specific embodiments of the present invention, the component (I) and the component (II) are in a weight ratio of 1:10-10:1.
In some specific embodiments of the present invention, the component (I) and the component (II) are in a weight ratio of 1:10-5:1.
In some specific embodiments of the present invention, the component (I) and the component (II) are in a weight ratio of 1:5-10:1.
In some specific embodiments of the present invention, the component (I) and the component (II) are in a weight ratio of 1:5-5:1.
In some specific embodiments of the present invention, the component (I) and the component (II) are in a weight ratio of 1:2.5-2.5:1.
In some specific embodiments of the present invention, the component (I) and the component (II) are in a weight ratio of 1:1-2.5:1.
In some specific embodiments of the present invention, the component (I) and the component (II) are in a weight ratio of 1:1, 5:1, 2.5:1, 1:2.5 and/or 1:5.
In a second aspect, the present invention further provides a drug comprising the composition and a pharmaceutically acceptable adjuvant.
In a third aspect, the present invention further provides use of the composition or the drug in the manufacture of a medicament for preventing and/or treating cerebrovascular diseases;

preferably, the cerebrovascular disease is selected from ischemic cerebrovascular disease;

preferably, the ischemic cerebrovascular disease is selected from ischemic stroke.

The pharmaceutical composition of the present invention can be used in the manufacture of a medicament for cerebrovascular diseases, wherein the cerebrovascular disease is preferably ischemic cerebrovascular disease, more preferably ischemic stroke.
The present invention has the following beneficial effects: according to the results of drug efficacy tests on animals (rats and mice), for cerebrovascular diseases, the combination of cilostazol and edaravone has the effect of synergistically increasing the drug efficacy.
The test results of the present invention show that the administration of cilostazol at 1-15 mg/kg or edaravone

at 1.67-8.33 mg/kg in the tail vein of rats with focal cerebral ischemia-reperfusion injury can significantly ameliorate the neurological deficits and reduce the area of cerebral infarction in MCAO rats; and the compounded combination (with a mass ratio of cilostazol: edaravone of 1:5-5:1) within the above dosage range can produce synergistic effect. The administration of cilostazol at 3.33-16.67 mg/kg or edaravone at 3.33-16.67 mg/kg in the tail vein of mice with focal cerebral ischemia-reperfusion injury can significantly ameliorate the neurological deficits and reduce the area of cerebral infarction in MCAO mice.

## DETAILED DESCRIPTION

The present invention discloses use of a composition comprising cilostazol in cerebrovascular diseases. Those skilled in the art can refer to the content of this article and appropriately improve the process parameters to realize the present invention. In particular, it should be noted that all similar replacements and modifications are apparent to those skilled in the art, and they are all considered to be included in the present invention. The method and use of the present invention have been described through preferred embodiments, and those skilled in the art can apparently make modifications or appropriate changes and combinations of the method and use described herein without departing from the content, spirit and scope of the present invention to realize and apply the technology of the present invention.

The raw materials and reagents used in the use of the composition comprising cilostazol in cerebrovascular diseases were all commercially available.

The present invention will be further illustrated below in conjunction with examples:

Example 1 Study on the protective effect of the composition of cilostazol and edaravone on focal cerebral ischemia-reperfusion injury 1

1 Materials and methods

1.1 Experimental animals

Sprague-Dawley (SD) rats, male, SPF-grade, weighing 250-280 g.

1.2 Test drugs

| Sample name | Manufacturer | Batch number |
|---|---|---|
| Cilostazol | Shanghai Aladdin Biochemical Technology | K1405071 |
| | Co.,Ltd. | |
| Edaravone | Jiangsu Tiansheng Pharmaceutical Co., Ltd. | 20161103 |

1.3 Experimental method

1.3.1 Animal grouping and administration

The experimental animals were divided into 4 groups, cilostazol group (1 mg/kg), edaravone group (5 mg/kg), cilostazol and edaravone composition group (6 mg/kg, cilostazol: edaravone=1:5, cilostazol 1 mg/kg+ edaravone 5 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

1.3.2 Establishment of focal cerebral ischemia-reperfusion model

The rat focal cerebral ischemia-reperfusion model was established by internal carotid artery suture method. The limbs (hind limbs above the knee joint and forelimbs below the wrist joint) and head of an anesthetized rat were tightened with rubber bands. The animal was fixed on an operating table in supine position, and was shaved with an animal shaver from the head to the chest, and the skin was disinfected with alcohol. The neck of the rat was cut at the midline, and the subcutaneous tissue was bluntly separated. The thin layer of fascia on the surface of the anterior triangle of the neck was separated, the lower side-lower edge of the clavicular hyoid muscle was pulled up, and the longitudinally pulsating artery parallel to this muscle can be seen. The arterial shell was opened, and the bifurcation of the right carotid artery was exposed. The right common carotid artery, external carotid artery and internal carotid artery were separated. The vagus nerve was gently stripped, and the external carotid artery was ligated and cut. The proximal end of the

common carotid artery was clamped. An incision was made at the distal end from the ligature of the external carotid artery, and was inserted with a suture line, which was passed through the bifurcation of the common carotid artery into the internal carotid artery, and then was inserted slowly until reaching slight resistance (approximately 20 mm from the bifurcation), so as to block all blood supply to the middle cerebral artery. The suture line was slightly fixed below the incision of the external carotid artery with silk thread. The silk thread clamping the proximal end of the common carotid artery was loosened. A gauze soaked in sterile saline was covered on the wound, and the rat was placed on a heat preservation pad to keep warm. 2.0 h after cerebral ischemia on the right side, the suture line was pulled out gently to restore the blood supply for reperfusion. The external carotid artery was ligated with silk thread fixing the suture line. The skin was stitched, and disinfected. The rats were placed in clean feed, and their general condition and respiration were observed until they woke up from anesthesia. The rats were provided with food and water, and commonly reared.

1.3.3 Determination of the area of cerebral infarction

The animals were subjected to evaluation of neurological deficit symptoms and then sacrificed with $CO_2$. The brain was taken out by cutting off head. The olfactory bulb, cerebellum and lower brainstem were removed. The blood on the surface of the brain was washed with normal saline, and the residual water on the surface was removed. The brain was placed at -20 °C for 20 min, then taken out, immediately cut to a coronal section vertically downward at the crossing plane of the line of sight, and sliced backward every 2 mm. The brain slices were incubated in 1% TTC staining solution (at 37 °C for 30 min). The normal brain tissue was stained into dark red, and the ischemic brain tissue was stained into pale white. After being washed with normal saline, the brain slices were quickly arranged in a row from front to back, removed off the residual water on the surface, and photographed.

Calculation of area of cerebral infarction: The photos were processed by Image J software, and the corresponding area of left brain and non-infarction area of right brain were calculated according to the formula, so that the percentage of the area of infarction was calculated.

Calculation of volume of infarction:

$$V=t\,(A1+A2+A3+\ldots\ldots\ldots+An),$$

t is the thickness of a slice, A is the area of infarction.

$$\%I=100\%\times(VC-VL)/VC,$$

%I is the percentage of volume of infarction, VC is the brain volume of the control side (left brain), and VL is the volume of the non-infarction area of the infarction side (right brain).

1.3.4 Analysis of synergy of composition

According to Jin Zhengjun's formula $q=E(a+b)/(Ea+Eb-Ea\times Eb)$, it was evaluated whether cilostazol and edaravone in the composition had a synergistic effect. In the formula, E(a+b) is the effective rate of combined drugs, and Ea and Eb are respectively the effective rates of drug A (cilostazol) and drug B (edaravone) alone. $E_{administration\ group} = (X_{model}-X_{administration})/X_{model}$, wherein X is the area of cerebral infarction. q value within the range of 0.85 to 1.15 represents a simple addition of the effect of the two drugs used, q value > 1.15 represents a synergistic effect, and q value < 0.85 represents that the combined use of the two drugs has an antagonistic effect.

1.4 Statistics

Experimental data were expressed as mean ± standard deviation (Mean ± SD). Differences among groups were analyzed by one-way analysis of variance, comparison between groups was tested by LSD method, and $P<0.05$ was defined as significant difference.

2 Experimental results

The effects on the area of cerebral infarction are shown in Table 1. The experimental results show that the administration of edaravone at 5 mg/kg of and the composition (cilostazol 1 mg/kg + edaravone 5 mg/kg) can significantly

reduce the area of cerebral infarction in animals (p=0.012, p=0.000), and the administration of cilostazol at 1 mg/kg tended to alleviate cerebral ischemic injury, but showed no statistical difference (p=0.08). The calculation result of synergy was q=1.33, indicating that the combined use of the two drugs had a synergistic effect.

Table 1 Effects of combined administration of cilostazol and edaravone on the area of cerebral infarction

| Group | Number of animals | Area of cerebral infarction (%) |
|---|---|---|
| Model group | 11 | 40.35±8.24 |
| Cilostazol group | 11 | 34.24 ± 7.25 |
| Edaravone group | 11 | 29.35±10.45* |
| Group of composition of cilostazol and edaravone in 1:5 | 12 | 19.74±9.30*** |
| Mean ± standard deviation, *p<0.05, *** p<0.001, compared with the model group. | | |

Example 2 Study on the protective effect of the composition of cilostazol and edaravone on focal cerebral ischemia-reperfusion injury 2

1 Materials and methods

1.1 Experimental animals

Sprague-Dawley (SD) rats, male, SPF-grade, weighing 250-280 g.

1.2 Test drugs

Cilostazol and edaravone were the same as in Example 1.

1.3 Experimental method

The experimental animals were divided into 4 groups, cilostazol group (5 mg/kg), edaravone group (5 mg/kg), cilostazol and edaravone composition group (10 mg/kg, cilostazol: edaravone=1:1, cilostazol 5 mg/kg+edaravone 5 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction, the analysis of synergy of the composition and the data statistics method were the same as in Example 1.

2 Experimental results

The effects on the area of cerebral infarction are shown in Table 2. The experimental results show that the administration of cilostazol at 5 mg/kg, edaravone at 5 mg/kg and the composition (cilostazol 5 mg/kg + edaravone 5 mg/kg) can significantly reduce the area of cerebral infarction in animals (p=0.025, p=0.008, p=0.000). The calculation result of synergy was q=1.47, indicating that the combined use of the two drugs had a synergistic effect.

Table 2 Effects of combined administration of cilostazol and edaravone on the area of cerebral infarction

| Group | Number of animals | Area of cerebral infarction (%) |
|---|---|---|
| Model group | 11 | 40.50±8.51 |
| Cilostazol group | 12 | 32.59 ±7.15* |
| Edaravone group | 11 | 29.49±9.06** |

(continued)

| Group | Number of animals | Area of cerebral infarction (%) |
|---|---|---|
| Group of composition of cilostazol and edaravone in 1:1 | 12 | 15.82±7.43*** |
| Mean ± standard deviation, *p<0.05, **p<0.01, *** p<0.001, compared with the model group. | | |

Example 3 Study on the protective effect of the composition of cilostazol and edaravone on focal cerebral ischemia-reperfusion injury 3

1 Materials and methods

1.1 Experimental animals

Sprague-Dawley (SD) rats, male, SPF-grade, weighing 250-280 g.

1.2 Test drugs

Cilostazol and edaravone were the same as in Example 1.

1.3 Experimental method

The experimental animals were divided into 4 groups, cilostazol group (15 mg/kg), edaravone group (3 mg/kg), cilostazol and edaravone composition group (18 mg/kg, cilostazol: edaravone=5:1, cilostazol 15 mg/kg+edaravone 3 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction, the analysis of synergy of the composition and the data statistics method were the same as in Example 1.

2 Experimental results

The effects on the area of cerebral infarction are shown in Table 3. The experimental results show that the administration of cilostazol at 15 mg/kg, edaravone at 3 mg/kg and the composition (cilostazol 15 mg/kg + edaravone 3 mg/kg) can significantly reduce the area of cerebral infarction in animals (p=0.007, p=0.029, p=0.000). The calculation result of synergy was q=1.38, indicating that the combined use of the two drugs had a synergistic effect.

Table 3 Effects of combined administration of cilostazol and edaravone on the area of cerebral infarction

| Group | Number of animals | Area of cerebral infarction (%) |
|---|---|---|
| Model group | 11 | 39.41±9.56 |
| Cilostazol group | 11 | 27.26 ± 9.74** |
| Edaravone group | 12 | 31.24±6.83* |
| Group of composition of cilostazol and edaravone in 5:1 | 12 | 17.08±6.26*** |
| Mean ± standard deviation, *p<0.05, **p<0.01, *** p<0.001, compared with the model group. | | |

Example 4 Effects of cilostazol/edaravone (1:5, 1:2.5, 1:1) on focal cerebral ischemia-reperfusion injury

1 Materials and methods

1.1 Experimental animals

Sprague-Dawley (SD) rats, male, SPF-grade, weighing 250-280 g.

1.2 Test drugs

Cilostazol and edaravone were the same as in Example 1.

1.3 Experimental method

The experimental animals were divided into 4 groups, a model group and three groups of compositions of cilostazol/edaravone (respectively a group of cilostazol 1.67 mg/kg+ edaravone 8.33 mg/kg in 1:5; a group of cilostazol 2.86 mg/kg+ edaravone 7.14 mg/kg in 1:2.5; a group of cilostazol 5 mg/kg+ edaravone 5 mg/kg in 1:1, and each composition was administered at a total amount of 10 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction and the data statistics method were the same as in Example 1.

2 Experimental results

The effects on the area of cerebral infarction are shown in Table 4. The experimental results show that the combined administration of cilostazol/edaravone in 1:5, 1:2.5 and 1:1 can significantly reduce the area of cerebral infarction in animals (p<0.001).

Table 4 Effects of compositions of cilostazol/edaravone on the area of cerebral infarction

| Group | Number of animals | Area of cerebral infarction (%) |
|---|---|---|
| Model group | 12 | 38.75±8.79 |
| Group of cilostazol/edaravone in 1:5 | 12 | 21.91 ±8.89*** |
| Group of cilostazol/edaravone in 1:2.5 | 12 | 19.46±9.39*** |
| Group of cilostazol/edaravone in 1:1 | 13 | 15.72±8.28*** |
| Mean ± standard deviation, ***p<0.001, compared with the model group. | | |

Example 5 Effects of cilostazol/edaravone (1:1, 2.5:1, 5:1) on focal cerebral ischemia-reperfusion injury

1 Materials and methods

1.1 Experimental animals

Sprague-Dawley (SD) rats, male, SPF-grade, weighing 250-280 g.

1.2 Test drugs

Cilostazol and edaravone were the same as in Example 1.

1.3 Experimental method

The experimental animals were divided into 4 groups, a model group and three groups of compositions of cilostazol/edaravone (respectively a group of cilostazol 5 mg/kg+ edaravone 5 mg/kg in 1:1; a group of cilostazol 7.14 mg/kg+ edaravone 2.86 mg/kg in 2.5:1; a group of cilostazol 8.33 mg/kg+ edaravone 1.67 mg/kg in 5:1, and each composition was administered at a total amount of 10 mg/kg). After the cerebral ischemia model was established, the

animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction and the data statistics method were the same as in Example 1.

2 Experimental results

The effects on the area of cerebral infarction are shown in Table 5. The experimental results show that the combined administration of cilostazol/edaravone in 1:1, 2.5:1 and 5:1 can significantly reduce the area of cerebral infarction in animals (p=0.000, p=0.000, p=0.001).

Table 5 Effects of compositions of cilostazol/edaravone on the area of cerebral infarction

| Group | Number of animals | Area of cerebral infarction (%) |
|---|---|---|
| Model group | 12 | 40.52±8.64 |
| Group of cilostazol/edaravone in 1:1 | 13 | 17.11±8.94*** |
| Group of cilostazol/edaravone in 2.5:1 | 12 | 22.90±11.37*** |
| Group of cilostazol/edaravone in 5:1 | 12 | 24.54±12.10** |

Mean ±standard deviation, **p<0.01, ***p<0.001, compared with the model group.

Example 6 Effects of cilostazol/edaravone (1:1, 1:2.5, 1:1) on focal cerebral ischemia-reperfusion injury in mice

1 Materials and methods

1.1 Experimental animals

C57BL/6J mice, male, SPF-grade, 8 week-old.

1.2 Test drugs

Cilostazol and edaravone were the same as in Example 1.

1.3 Experimental method

The experimental animals were divided into 4 groups, a model group and three groups of compositions of cilostazol/edaravone (respectively a group of cilostazol 3.33 mg/kg+ edaravone 16.67 mg/kg in 1:5; a group of cilostazol 5.71 mg/kg+ edaravone 14.29 mg/kg in 1:2.5; a group of cilostazol 10 mg/kg+ edaravone 10 mg/kg in 1:1, and each composition was administered at a total amount of 20 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

Establishment of focal cerebral ischemia-reperfusion model: The mouse focal cerebral ischemia-reperfusion model was established by internal carotid artery suture method. An anesthetized mouse was fixed on an operating table in a supine position. The neck of the mice was cut at the midline, and the subcutaneous tissue was bluntly separated. The right common carotid artery, external carotid artery, and internal carotid artery were separated. The external carotid artery was inserted with a suture line, which was passed through the bifurcation of the common carotid artery into the internal carotid artery, and then was inserted slowly until reaching slight resistance (approximately 10 mm from the bifurcation), so as to block all blood supply to the cerebral artery. 60 minutes after cerebral ischemia on the right side, the suture line was gently pulled out to restore the blood supply for reperfusion. The mice were placed in clean feed, and their general condition and respiration were observed until they woke up from anesthesia. The mice were provided with food and water, and commonly reared.

The determination of the area of cerebral infarction and the data statistics method were the same as in Example 1.

2 Experimental results

The effects on the area of cerebral infarction are shown in Table 6. The experimental results show that the combined administration of cilostazol/edaravone in 1:5, 1:2.5 and 1:1 can significantly reduce the area of cerebral infarction in animals (p<0.001, p<0.012, p<0.001).

Table 6 Effects of compositions of cilostazol/edaravone on the area of cerebral infarction

| Group | Number of animals | Area of cerebral infarction (%) |
|---|---|---|
| Model group | 12 | 36.24±9.46 |
| Group of cilostazol/edaravone in 1:5 | 13 | 22.49 ±8.76*** |
| Group of cilostazol/edaravone in 1:2.5 | 12 | 21.12±11.04** |
| Group of cilostazol/edaravone in 1:1 | 13 | 16.75±8.44*** |

Mean ± standard deviation, ** p<0.01, *** p<0.001, compared with the model group.

Example 7 Effects of cilostazol/edaravone (1:1, 2.5:1, 5:1) on focal cerebral ischemia-reperfusion injury in mice

1 Materials and methods

1.1 Experimental animals

C57BL/6J mice, male, SPF-grade, 8 week-old.

1.2 Test drugs

Cilostazol and edaravone were the same as in Example 1.

1.3 Experimental method

The experimental animals were divided into 4 groups, a model group and three groups of compositions of cilostazol/edaravone (respectively a group of cilostazol 10 mg/kg+ edaravone 10 mg/kg in 1:1; a group of cilostazol 14.29 mg/kg+ edaravone 5.71 mg/kg in 2.5:1; a group of cilostazol 16.67 mg/kg+ edaravone 3.33 mg/kg in 5:1, and each composition was administered at a total amount of 20 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of focal cerebral ischemia-reperfusion model was the same as in Example 6, and the determination of the area of cerebral infarction and the data statistics method were the same as in Example 1.

2 Experimental results

The effects on the area of cerebral infarction are shown in Table 7. The experimental results show that the combined administration of cilostazol/edaravone in 1:1, 2.5:1 and 5:1 can significantly reduce the area of cerebral infarction in animals (p<0.001).

Table 7 Effects of compositions of cilostazol/edaravone on the area of cerebral infarction

| Group | Number of animals | Area of cerebral infarction (%) |
|---|---|---|
| Model group | 13 | 39.06±7.90 |
| Group of cilostazol/edaravone in 1:1 | 13 | 15.58±8.95*** |
| Group of cilostazol/edaravone in 2.5:1 | 12 | 21.23±10.84*** |

(continued)

| Group | Number of animals | Area of cerebral infarction (%) |
|---|---|---|
| Group of cilostazol/edaravone in 5:1 | 12 | 23.88±11.90*** |
| Mean ± standard deviation, ***p<0.001, compared with the model group. | | |

The use of a composition comprising cilostazol in cerebrovascular diseases provided by the present invention has been described in detail above. The principle and embodiments of the present invention are illustrated herein by using specific examples. The description of the above examples is only used to help understand the method and core idea of the present invention. It should be noted that for those skilled in the art, without departing from the principle of the present invention, several improvements and modifications can be made to the present invention, and these improvements and modifications also fall within the protection scope of the claims of the present invention.

## Claims

**1.** A composition comprising the following components:

component (I), cilostazol, a derivative, a pharmaceutically acceptable salt, or a prodrug molecule thereof; and component (II), edaravone, or a drug with an active ingredient of edaravone.

**2.** The composition according to claim 1, wherein the component (I) and the component (II) are in a weight ratio of 1:10-10:1.

**3.** The composition according to claim 1, wherein the component (I) and the component (II) are in a weight ratio of 1:10-5:1.

**4.** The composition according to claim 1, wherein the component (I) and the component (II) are in a weight ratio of 1:5-10:1.

**5.** The composition according to claim 1, wherein the component (I) and the component (II) are in a weight ratio of 1:5-5:1.

**6.** The composition according to claim 1, wherein the component (I) and the component (II) are in a weight ratio of 1:2.5-2.5:1.

**7.** The composition according to claim 1, wherein the component (I) and the component (II) are in a weight ratio of 1:1-2.5:1.

**8.** The composition according to claim 1, wherein the component (I) and the component (II) are in a weight ratio of 1:1, 5:1, 2.5:1, 1:2.5 and/or 1:5.

**9.** A drug, comprising the composition according to any one of claims 1 to 8, and a pharmaceutically acceptable adjuvant.

**10.** Use of the composition according to any one of claims 1 to 8 or the drug according to claim 9 in the manufacture of a medicament for preventing and/or treating cerebrovascular diseases; preferably, the cerebrovascular disease is selected from ischemic cerebrovascular disease; preferably, the ischemic cerebrovascular disease is selected from ischemic stroke.

## INTERNATIONAL SEARCH REPORT

International application No.
**PCT/CN2022/073910**

**A. CLASSIFICATION OF SUBJECT MATTER**

A61K 31/4709(2006.01)i; A61K 31/4152(2006.01)i; A61P 9/10(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT, CNABS, CNKI, VEN, DWPI, WOTXT, USTXT, PUBMED, STN, ISI Web of Science, 万方数据, 南京宁丹新药技术有限公司, 王磊, 张正平, 陈荣, 杨士豹, 西洛他唑, 依达拉奉, 卒中, 梗塞, 梗死, 缺血, cilostazol, vinpocetine, PDE3, phosphodiesterase, edaravone, cerebrovascular, stroke, ischemia, infarction, infarct, 73963-72-1, 89-25-8

**C. DOCUMENTS CONSIDERED TO BE RELEVANT**

| Category* | Citation of document, with indication, where appropriate, of the relevant passages | Relevant to claim No. |
|---|---|---|
| X | YAMAMOTO, Yasumasa et al.,. "A Combined Treatment for Acute Larger Lacunar-Type Infarction," *Journal of Stroke and Cerebrovascular Diseases,* Vol. 20, No. 5, 31 October 2011 (2011-10-31), pp. 387-394 | 1-10 |
| A | OHTA, Yasuyuki et al.,. "Efficacy of the Free Radical Scavenger, Edaravone, for Motor Palsy of Acute Lacunar Infarction," *INTERNAL MEDICINE,* Vol. 48, 31 December 2009 (2009-12-31), pp. 593-596 | 1-10 |
| A | KIMURA, Teruo et al.,. "Ultra-Early Combination Antiplatelet Therapy with Cilostazol for the Prevention of Branch Atheromatous Disease: A Multicenter Prospective Study," *Cerebrovasc Dis Extra,* Vol. 6, 12 October 2016 (2016-10-12), pp. 84-95 | 1-10 |
| A | IKEDA, Satoshi et al.,. "Effects of Edaravone, a Free Radical Scavenger, on Photochemically Induced Cerebral Infarction in a Rat Hemiplegic Model," *The Scientific World Journal,* 31 December 2013 (2013-12-31), pp. 1-5 | 1-10 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 March 2022** | **01 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2022/073910

C. DOCUMENTS CONSIDERED TO BE RELEVANT

| Category* | Citation of document, with indication, where appropriate, of the relevant passages | Relevant to claim No. |
|---|---|---|
| A | 吴云 等 (WU, Yun et al.). "西洛他唑对糖氧剥离大鼠脑微血管内皮细胞的保护作用 (Protection of Cilostazol on Cultured Brain Microvascular Endothelial Cells Deprived of Oxygen and Glucose in Rats)" *中风与神经疾病杂志 (Journal of Apoplexy and Nervous Diseases),* Vol. 27, No. 10, 31 October 2010 (2010-10-31), pp. 898-901 | 1-10 |
| A | US 2009297596 A1 (ELAN PHARMA INTERNATIONAL LIMITED) 03 December 2009 (2009-12-03) entire description | 1-10 |

Form PCT/ISA/210 (second sheet) (January 2015)

**INTERNATIONAL SEARCH REPORT**
**Information on patent family members**

International application No.
**PCT/CN2022/073910**

| Patent document cited in search report | | | Publication date (day/month/year) | Patent family member(s) | | | Publication date (day/month/year) |
|---|---|---|---|---|---|---|---|
| US | 2009297596 | A1 | 03 December 2009 | BR | PI0609982 | A2 | 18 May 2010 |
| | | | | EA | 200702595 | A1 | 30 December 2008 |
| | | | | ZA | 200710000 | B | 30 December 2009 |
| | | | | IL | 187567 | D0 | 01 August 2011 |
| | | | | NO | 20076588 | L | 20 February 2008 |
| | | | | JP | 2008545808 | A | 18 December 2008 |
| | | | | WO | 2008030209 | A2 | 13 March 2008 |
| | | | | WO | 2008030209 | A3 | 03 July 2008 |
| | | | | CA | 2611506 | A1 | 23 November 2006 |
| | | | | EP | 1937218 | A2 | 02 July 2008 |
| | | | | CN | 101287451 | A | 15 October 2008 |
| | | | | KR | 20080047509 | A | 29 May 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110122555